# EUROPÄISCHE PATENTANMELDUNG

(11) **EP 1 498 092 A1**
(43) Veröffentlichungstag der Anmeldung: **19.01.2005**
(21) Anmeldenummer: 04405422.9
(22) Anmeldetag: 06.07.2004
(51) Int. Cl.: A61F 11/08

(54) **Gehörschutz**

(30) Priorität: 14.07.2003 CH 12252003
(71) Anmelder: Poissenot, Ruth, 4058 Basel (CH)
(72) Erfinder: Poissenot, Ruth, 4058 Basel (CH)
(74) Vertreter: Heinen, Detlef

(57) **Zusammenfassung**

Ein Gehörschutz umfasst eine in den Gehörgang eines Benutzers einsetzbare Otoplastik (1) und einen in die Otoplastik (1) in einer vordefinierten Position eingesetzten Filtereinsatz (2). Der Filtereinsatz (2) und die Otoplastik (1) sind ist so ausgebildet, dass der Filtereinsatz (2) durch den Benutzer selbst aus der Otoplastik (1) entfernbar und wieder korrekt in die vordefinierte Position einsetzbar ist.

## Beschreibung

Die Erfindung betrifft einen Gehörschutz gemäss dem Oberbegriff des unabhängigen Patentanspruchs 1.

Ein solcher Gehörschutz wird beispielsweise dazu verwendet das Gehör eines Benutzers vor einer Lärmbelastung, beispielsweise vor Baulärm, zu schützen. Dabei soll einerseits der Lärm, beispielsweise durch einen Presslufthammer verursacht, selektiv durch den Gehörschutz gedämpft werden und andererseits gleichzeitig erwünschter Schall, wie beispielsweise gesprochene Sprache, vom Benutzer wahrgenommen werden können. Dazu werden üblicherweise Filter eingesetzt, die in verschiedenen Frequenzereichen eine unterschiedliche Dämpfung aufweisen.

Es sind schon verschiedene Gehörschutztypen vorgeschlagen worden. Beispielsweise wird in CH-A-254,103 ein Gehörschutz in Form eines in das Ohr einsteckbaren Pfropfens beschrieben. Der Pfropfen ist an seinem dem Gehörgang zugewandten Ende mit einer flexiblen Hülle ausgestattet, die in der Lage ist, sich von der Form her gut an den Gehörgang anzupassen, um ihn zu verschliessen. Der Pfropfen weist ein zweigliedriges Tiefpassfilter auf, welches für Frequenzen im tiefen bis mittleren Bereich (also insbesondere für gesprochene Sprache) relativ gut durchlässig ist, wohingegen höhere Frequenzen stark gedämpft bzw. praktisch nicht durchgelassen werden. Das zweigliedrige Tiefpassfilter ist in mehreren Ausführungsbeispielen vorgeschlagen, wobei allgemein einem Hohlraum ein verhältnismässig enger Kanal vorgeschaltet ist. Die Länge des Kanals und des Hohlraums ist im Verhältnis zu der abzuschirmenden Wellenlänge klein. Der Hohlraum ist durch eine akustische Impedanz abgeschlossen, die dem Wellenwiderstand der durchzulassenden Frequenzen ungefähr angepasst ist.

In einem weiteren Vorschlag zur Realisierung eines Gehörschutzes zeigt die US-A-4,353,364 einen Gehörschutz in Form eines Stopfens. Der Stopfen weist einen z.B. sechseckigen aussenliegenden Grundkörper auf und einen gehörgangseitigen, sich verjüngenden Teil. Dieser Teil kann spiralförmig sich um den Schaft windende Stege aufweisen, welche beim Einführen des Stopfens in den Gehörgang den Gehörgang verschliessen. Ferner ist ein Schallkanal vorgesehen, welcher sich vom äusseren Ende des Stopfens durch den gesamten Stopfen bis zum gehörgangseitigen Ende erstreckt. Der Schallkanal kann seinen Durchmesser verringern und danach ggf. wieder erweitern. Durch die Verringerung des Durchmessers wird der Schallpegel reduziert. In dem Stopfen ist ein Sackloch vorgesehen, in welchem ein Stöpsel lösbar eingeklemmt gehalten wird. Das Sackloch weist gegenüber dem Stöpsel bzw. Teilen davon ein Untermass auf, sodass der Stöpsel in dem Sackloch eingequetscht gehalten wird. Der nach aussen weisende Teil des Schallkanals hat den gleichen Durchmesser wie das Sackloch, so dass zum Verschliessen des Schallkanals der Stöpsel aus dem Sackloch herausgezogen und in den nach aussen weisenden Teil des Schallkanals eingesetzt werden kann, wo er dann eingequetscht gehalten wird.

Ein weiterer Gehörschutz ist in der US-A-2,717,596 vorgeschlagen. In dieser ist ein Gehörschutz mit einer äusseren Hülle aus einem flexiblen Material wie Gummi oder dergleichen beschrieben. Die Hülle umfasst eine nach aussen weisende in Richtung auf den Gehörgang zu konisch ausgebildete Kammer, in welche ein ebenfalls konisch ausgebildeter Einsatz eingesetzt ist. Der Einsatz besteht beispielsweise aus Blei oder einem anderen Schwermetall und weist eine durchgehende Längsbohrung mit kleinem Durchmesser auf. Die Seitenwand des Einsatzes verläuft in Richtung auf den Gehörgang zu konisch, sodass der Einsatz nach dem Einbringen in die Kammer an der Seitenwand durch Reibung festsitzt. Im betriebstauglichen Zustand ist der Einsatz vollständig innerhalb der Kammer angeordnet und durch den Benutzer nicht mehr ohne weiteres entfernbar. An dem gehörgangseitigen Ende weist die Kammer eine zentrale Durchgangsbohrung auf, die den gehörgangseitigen Boden der Kammer bildet. Ausserdem ist der Gehörschutz gehörgangseitig mit einem ringförmigen, wulstartigen Flansch versehen, der in den Gehörgang eingebracht wird und diesen verschliesst, so dass der Schall nur durch die Längsbohrung des Einsatzes und durch die Durchgangsbohrung in den Gehörgang gelangen kann.

In einem weiteren Vorschlag zeigt die US-A-2,881,759 einen für den Benutzer individuell hergestellten Gehörschutz, der im Prinzip zweiteilig ist. Er umfasst einen Grundkörper, der speziell an das Ohr (Gehörgang sowie Aussenohr) des Benutzers angepasst ist. In diesem Grundkörper ist eine nach aussen weisende Aussparung vorgesehen. In der Seitenwand dieser Aussparung ist ein Kanal mit sehr kleinem Durchmesser vorgesehen, welcher den in den Kanal eintretenden Schall durch den Grundkörper hindurch in den Gehörgang leitet. In der Aussparung ist ein Schieber angeordnet, welcher in einem bestimmten Bereich der Aussparung verschiebbar ist. Die Aussparung ist am gehörgangsseitigen Ende geringfügig hinterschnitten ausgebildet und klemmt durch die elastischen Eigenschaften des Materials den zylindrischen Schieber, sobald das vordere Ende des zylindrischen Schiebers die Hinterschneidung passiert hat. In diesem Zustand verschliesst der Schieber den in der Seitenwand der Aussparung vorgesehenen Kanal. Der Schieber ist nach aussen hin gegen zufälliges Verlieren gesichert in der Aussparung angeordnet, indem diese einen ringförmig nach innen vorstehenden Vorsprung aufweist. Der Schieber befindet sich jedoch nicht gesichert in einer bestimmten Position, was unstete Dämpfungseigenschaften zur Folge haben kann.

Die FR-A-2,039,825 schlägt schliesslich einen zweiteiligen pfropfenartigen Gehörschutz vor. Der Gehörschutz umfasst einen äusseren Stopfen, der aus einem flexiblen Material besteht und eine sich längs durch den Stopfen erstreckende Bohrung aufweist. Der Stopfen weist einen aussenliegenden Wulst auf. Beim Einführen des Stopfens in den Gehörgang wird dieser so weit eingeführt bis der Wulst an der entsprechenden Gegenfläche am menschlichen Ohr anliegt. In der sich längs durch den Stopfen erstreckenden Bohrung ist ein Filterelement angeordnet, welches in Richtung zum Gehörgang hin betrachtet Eingangsabschnitte sowie einen daran anschliessenden Kanal aufweist. Gegen das gehörgangseitige Ende des Kanals hin reduziert sich dessen Durchmesser stark. Je nach Verhältnis der beiden Kanaldurchmesser zueinander kann die Filterwirkung verändert werden. Das Filterelement ist an seinem dem Gehörgang zugewendeten Ende pfeilförmig ausgebildet, wodurch beim Einbringen des Filterelements das Material des Stopfens hinter das pfeilförmige Ende des Filterelements "fliesst" und dadurch das Filterelement fest mit dem Stopfen verbindet.

Die beschriebenen Gehörschutztypen sind jeweils für ganz bestimmte Einsatzzwecke mit einer speziell für diesen Zweck filternden Konstruktion ausgestaltet. Entweder sind solche Filterkonstruktionen gar nicht oder ausschliesslich durch Fachpersonal auswechselbar bzw. anpassbar oder sie haben keine vordefinierten Dämpfungseigenschaften, was ein häufiges Anpassen nötig machen kann aber jedenfalls keine konstante gewünschte Dämpfung gewährleistet. Werden Gehörschutze für mehrere verschiedene Einsatzzwecke mit vordefinierten fixen Dämpfungseigenschaften benötigt, muss der Benutzer entweder mehrere Gehörschutze besitzen, was insbesondere bei massgefertigten Gehörschutzen teuer ist, oder er muss die filternden Konstruktionen durch Fachpersonal wechseln lassen, was sehr aufwändig ist.

Aufgabe der nachfolgenden Erfindung ist es daher, einen Gehörschutz vorzuschlagen, welcher einerseits stete, spezifische und konstante Dämpfungseigenschaften aufweist und andererseits für verschiedene Einsatzzwecke geeignet ist, ohne dabei die vorstehend erwähnten Nachteile aufzuweisen.

Diese Aufgabe wird erfindungsgemäss durch einen Gehörschutz gelöst, wie er durch die Merkmale des unabhängigen Patentanspruchs 1 charakterisiert ist. Vorteilhafte Ausgestaltungen des erfindungsgemässen Gehörschutzes ergeben sich aus den Merkmalen der abhängigen Patentansprüche.

Insbesondere umfasst der erfindungsgemässe Gehörschutz eine Otoplastik und einen Filtereinsatz, welche so ausgebildet sind, dass der in die Otoplastik in eine vordefinierten Position eingesetzte Filtereinsatz durch den Benutzer selbst aus der Otoplastik entfernbar und wieder korrekt in die vordefinierte Position einsetzbar ist. Ein solcher Gehörschutz zeichnet sich dadurch aus, dass eine einzige Otoplastik, die massgefertigt sein kann, bedarfsweise vom Benutzer selbst mit einem der jeweiligen Lärmsituation angepassten Filter versehen werden kann, sodass der Gehörschutz auf sehr einfache Weise vom Benutzer selbst so konfektioniert werden kann, dass er für den jeweiligen Einsatzzweck angepasste, stete und konstante Dämpfungseigenschaften aufweist.

Der Filtereinsatz kann eine Filterhülse umfassen, die aus einem Material hergestellt ist, z. B. aus Polyethylen niedriger Dichte (LDPE), welches relativ zu dem Material, aus dem die Otoplastik hergestellt ist, z. B. Polyacryl, quasi nicht verformbar ist, sowie ein in die Filterhülse eingesetztes Filter. Eine solche Filterhülse sitzt nach dem Einsetzen über die durch die Verformung der Otoplastik wirkenden Kräfte in der Otoplastik fest. Ausserdem schützt sie das Filter vor mechanischer Beanspruchung und gewährleistet dessen wohldefinierte Schalldämpfungswirkung.

Der Filtereinsatz kann einen Rand umfassen, der nach aussen hin absteht. Ein solcher Filtereinsatz kann beim Einsetzen in die Otoplastik eingeschoben werden bis der Rand an der Otoplastik anliegt, was gewährleistet, dass das Filter genau in die vordefinierte Position eingesetzt wird.

Der Filtereinsatz kann einen kegelstumpfförmigen Bereich aufweisen. Ein solche Ausführungsform ermöglicht ein einfaches Einsetzen des Filtereinsatzes in die Otoplastik, wie oben erwähnt.

Der Rand des Filtereinsatzes kann am distalen Ende des kegelstumpfförmigen Bereichs angeordnet sein. Ein solcher Filtereinsatz wird beim Einsetzen über den ganzen kegelstumpfförmigen Bereich hinweg in die Otoplastik eingeführt, was eine gutes Festsitzen des Filtereinsatzes in der Otoplastik bewirkt.

Die Otoplastik kann individuell dem Gehörgang seines Benutzers angepasst sein. Dadurch kann eine gute Abdichtung zwischen Gehörgang und Gehörschutz bei gleichzeitig hohem Tragekomfort erreicht werden.

Die Otoplastik kann lediglich den Eingangsbereich des Gehörgangs abdecken, was den Tragekomfort weiter erhöht und ästhetisch vorteilhaft ist.

Die Otoplastik kann Mittel zum Einsetzen des Gehörschutzes in den Gehörgang bzw. zum Entfernen des Gehörschutzes aus dem Gehörgang umfassen, beispielsweise einen halbringförmigen Fortsatz. Mit solchen Mitteln ist der Gehörschutz auf einfache Weise vom Benutzer in den Gehörgang einsetzbar bzw. entfernbar.

Ein weiterer Aspekt der Erfindung betrifft den Filtereinsatz des erfindungsgemässen Gehörschutzes. Dieser weist, wie vorstehend schon erwähnt, in vorteilhafter Weise einen kegelstumpfförmigen Bereich und einen am distalen Ende des kegelstumpfförmigen Bereichs angeordneten Rand auf, der von dem kegelstumpfförmigen Bereich nach aussen hin absteht. Ein Filtereinsatz mit einem solchen kegelstumpfförmigen Bereich ermöglicht ein einfaches Einsetzen des Filtereinsatzes in die Otoplastik. Ausserdem kann er beim Einsetzen in die Otoplastik eingeschoben werden bis der Rand an der Otoplastik anliegt. Dadurch ist gewährleistet, dass das Filter in die vordefinierte Position eingesetzt wird.

Der Filtereinsatz kann eine Filterhülse, beispielsweise aus Polyethylen niedriger Dichte (LDPE), und einen in die Filterhülse eingesetztes Filter umfassen. Durch eine solche Filterhülse wird das Filter vor mechanischer Beanspruchung geschützt und dessen wohldefinierte Dämpfungswirkung ist gewährleistet.

Noch ein weiterer Aspekt der Erfindung betrifft ein Filtereinsatzset, das mindestens zwei erfindungsgemässe Filtereinsätze mit unterschiedlichen Dämpfungseigenschaften umfasst. Solche Filtereinsatzsets werden zusammengestellt, um angepassten Gehörschutz für unterschiedliche Einsatzbereiche anzubieten.

Weitere vorteilhafte Ausgestaltungen der Erfindung ergeben sich aus der nachfolgenden Beschreibung von Ausführungsbeispielen der Erfindung mit Hilfe der schematischen Zeichnung.

Es zeigen:
- Fig. 1: einen Schnitt durch ein Ausführungsbeispiel eines erfindungsgemässen Gehörschutzes,
- Fig. 2: eine Ansicht eines ersten Ausführungsbeispiels einer Filterhülse des erfindungsgemässen Gehörschutzes,
- Fig. 3: einen Schnitt durch die Filterhülse aus Fig. 2,
- Fig. 4: eine Ansicht eines zweiten Ausführungsbeispiels einer Filterhülse des erfindungsgemässen Gehörschutzes,
- Fig. 5: einen Schnitt durch die Filterhülse aus Fig. 4,
- Fig. 6: eine Ansicht eines Werkzeugs zum Entnehmen des Filtereinsatzes des erfindungsgemässen Gehörschutzes,
- Fig. 7: einen Schnitt durch ein Ausführungsbeispiel eines Filters eines erfindungsgemässen Gehörschutzes,
- Fig. 8: einen Schnitt durch ein zweites Ausführungsbeispiel eines Filters des erfindungsgemässen Gehörschutzes,
und
- Fig. 9: eine graphische Darstellung von Schalldämpfungskurven zweier verschiedener Filter des erfindungsgemässen Gehörschutzes.

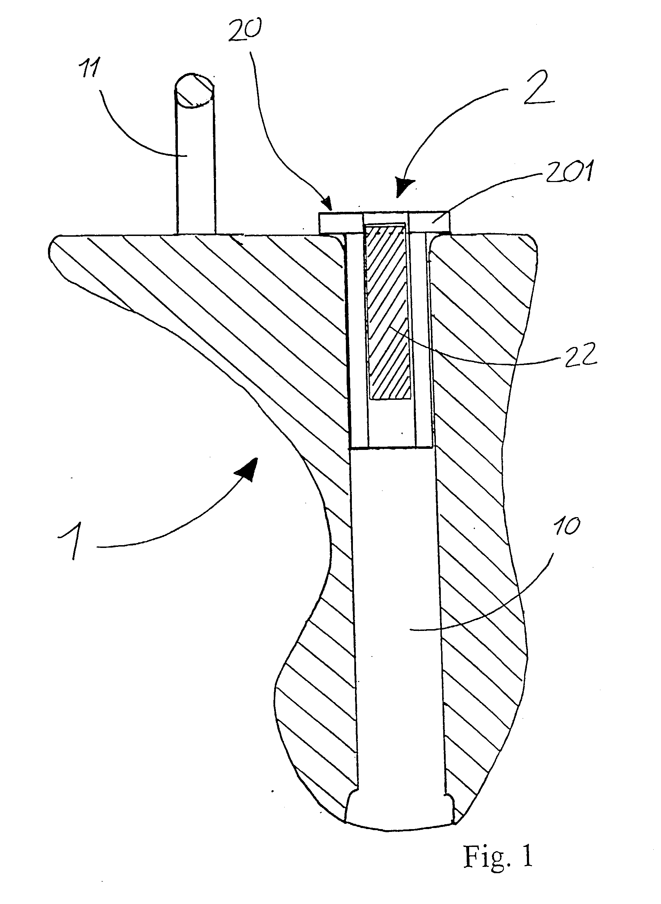

In Fig. 1 ist ein Ausführungsbeispiel eines erfindungsgemässen Gehörschutzes dargestellt. Der Gehörschutz umfasst eine Otoplastik 1 und einen Filtereinsatz 2. Die Otoplastik 1 ist von der Form her individuell der Form des Gehörgangs eines Benutzers angepasst und aus einem verformbaren Material hergestellt. Nach dem Einsetzen in den Gehörgang schliesst die Otoplastik 1 an ihrem äusseren Umfang den Gehörgang des Benutzers schalldicht ab und deckt den Eingangsbereich des Gehörgangs ab. An ihrem distalen, d.h. dem Innenohr und somit dem Benutzer abgewandten Ende, weist die Otoplastik 1 Mittel auf, z.B. einen halbringförmigen Fortsatz 11, mit denen der Gehörschutz einfach in den Gehörgang eingesetzt bzw. aus dem Gehörgang entfernt werden kann.

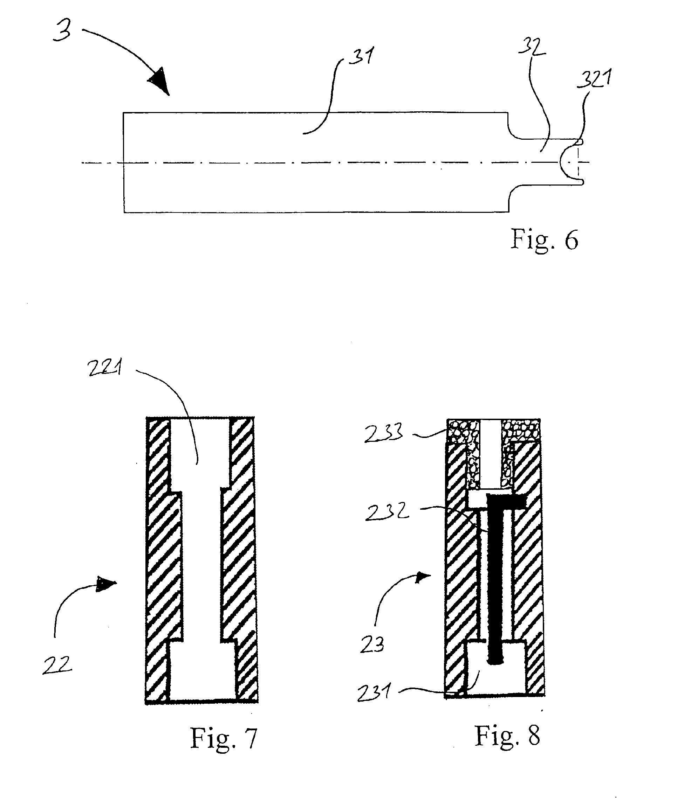

Ausserdem weist die Otoplastik 1 einen den Gehörgang mit der Aussenwelt verbindenden Durchgang 10 auf, in den der Filtereinsatz 2 so eingesetzt ist, dass einerseits mit Hilfe eines geeigneten Werkzeuges (s. Fig. 6) durch den Benutzer ausgewechselt werden kann und andererseits die Otoplastik 1 schalldicht verschliesst. Der Filtereinsatz 2 umfasst eine Filterhülse 20 mit einem nach aussen vorstehenden Rand 201, in die ein Filter 22 eingesetzt ist, durch welches kontrolliert gefilterter und gedämpfter Schall dem Gehör des Benutzers zugeleitet wird.

Das verformbare Material der Otoplastik 1, z.B. Polyacryl, weist in Bezug auf Reizungen und Entzündungen der Haut des Benutzers vorteilhafte Eigenschaften auf. Die Filterhülse 20 ist aus einem Material hergestellt, welches relativ zum Material der Otoplastik 1 quasi nicht verformbar ist, insbesondere aus Polyethylen niedriger Dichte (LDPE). Zum Einsetzen des Filtereinsatzes 2 in die Otoplastik 1 wird dieser soweit in den Durchgang 10 eingeführt bis der Rand 201 der Filterhülse 20 auf der Otoplastik 1 aufliegt. Dabei wird die Otoplastik 1 im Kontaktbereich mit dem Filtereinsatz 2 so verformt, dass sie diesen in einer korrekten vorbestimmten Position festklemmt und vor einem ungewollten Entfernen schützt. Diese Klemmung ist aber nur so gross, dass der Filtereinsatz 2 ohne Beschädigung der Otoplastik 1 und des Filtereinsatzes 2 auch wieder entfernt werden kann. So kann je nach Einsatzgebiet der Gehörschutz mit einem entsprechenden Filtereinsatz 2 versehen werden. Vorteilhafterweise besitzt der Benutzer ein Set von Filtereinsätzen für die verschiedenen Einsatzgebiete, in denen er den Gehörschutz benötigt.

Für die gesamte weitere Beschreibung gilt folgende Festlegung. Sind im Beschreibungstext einer Figur Bezugszeichen enthalten, die nicht in der unmittelbar zugehörigen Figur enthalten sind, so wird auf deren Erläuterung in vorangehenden Figurenbeschreibungen Bezug genommen.

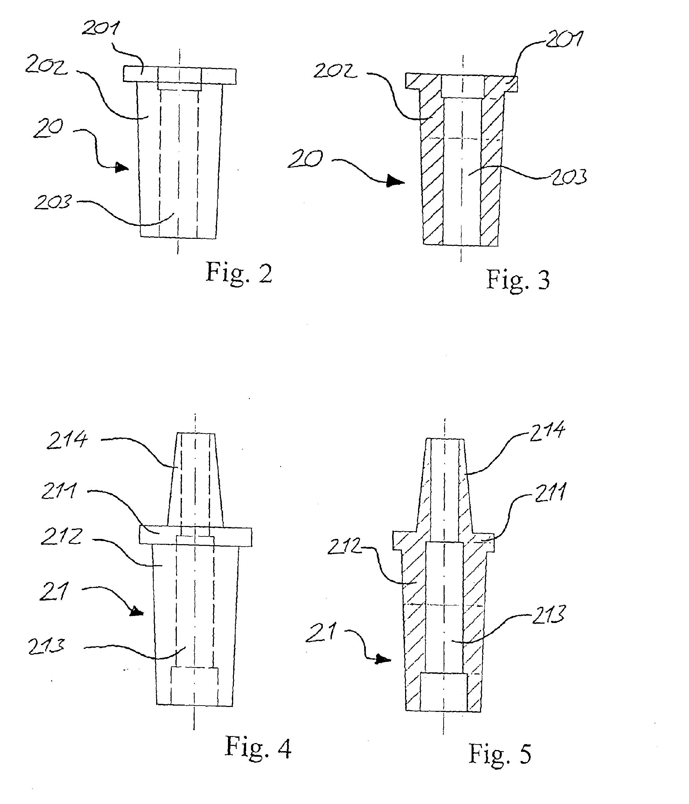

Fig. 2 und Fig. 3 zeigen eine Ansicht eines ersten Ausführungsbeispiels bzw. einen Längsschnitt durch ein erstes Ausführungsbeispiel einer Filterhülse 20 des erfindungsgemässen Gehörschutzes. Die Filterhülse 20 umfasst einen kegelstumpfförmigen Bereich 202, an dessen breiterem Ende der Rand 201 angeordnet ist. Dieser weist einen grösseren äusseren Durchmesser auf als der grösste äussere Durchmesser des kegelstumpfförmigen Bereichs 202 und steht somit nach aussen ab. Im Zentrum der Filterhülse 20 ist ein Filterdurchgang 203 angeordnet, in welchem ein Filter 22 fest angeordnet sein kann. Der kegelstumpfförmige Bereich 202 dient dazu, das Einführen des Filtereinsatzes 2 in die Otoplastik 1 zu erleichtern und eine Verklemmung des Filtereinsatzes 2 mit der Otoplastik 1 zu gewährleisten. Durch die Wahl eines Materials zur Herstellung der Filterhülse 20, welches relativ zum Material der Otoplastik 1 quasi nicht verformbar ist, insbesondere Polyethylen niedriger Dichte (LDPE), findet die Verklemmung im Prinzip durch eine Verformung der Otoplastik 1 statt. Zusätzlich ist mit der Wahl eines solchen Materials das Filter 22 im Inneren der Filterhülse 20 vor mechanischer Beanspruchung beim Einsetzten und Entnehmen des Filtereinsatzes 2 geschützt.

Fig. 4 und Fig. 5 zeigen eine Ansicht eines zweiten Ausführungsbeispiels bzw. einen Längsschnitt durch ein zweites Ausführungsbeispiels einer Filterhülse 21 des erfindungsgemässen Gehörschutzes. Entsprechend der Filterhülse 20 aus Fig. 2 und Fig. 3 ist bei der Filterhülse 21 ein kegelstumpfförmiger Bereich 212, ein Rand 211 und ein Filterdurchgang 213 angeordnet. Zusätzlich umfasst die Filterhülse 21 einen Stutzen 214. Der Stutzen 214 ist kegelstumpfförmig ausgestaltet und mit seinem breiteren Ende an der dem kegelstumpfförmigen Bereich 212 entgegengesetzten Seite des Randes 211 angeordnet. Er dient dazu, Lecktests durchzuführen.

In Fig. 6 ist ein Werkzeug 3 zum Entnehmen des Filtereinsatzes 2 des erfindungsgemässen Gehörschutzes dargestellt. Das Werkzeug 3 umfasst einen Hebelbereich 31 und einen an dessen einen Längsende angeordneten Werkzeugkopf 32. Der Werkzeugkopf 32 umfasst an seinem dem Hebelbereich 31 abgewandten Ende eine halbkreisförmige Aussparung 321 mit einem Radius, der vorzugsweise etwas grösser als der grösste Radius des kegelstumpfförmigen Bereichs 202 bzw. 212 und kleiner als der Radius des Randes 201 bzw. 211 der zu bedienenden Filterhülse 20 bzw. 21 ist. Zum Entnehmen des Filtereinsatzes 2 aus der Otoplastik 1 wird die Aussparung 321 zwischen dem Rand 201 bzw. 211 und der Otoplastik 1 angesetzt, sodass der Werkzeugkopf 32 den kegelstumpfförmige Bereich 202 bzw. 212 unterhalb des Randes 201 bzw. 211 umgreift. Über den Hebelbereich 31 kann der Benutzer das Werkzeug 3 so betätigen, dass sich der Filtereinsatz 2 aus der Otoplastik 1 löst und entfernt werden kann.

In Fig. 7 ist ein erstes Ausführungsbeispiel eines Filters 22 gezeigt, wie es im Filtereinsatz 2 des erfindungsgemässen Gehörschutzes eingesetzt sein kann. Das Filter 22 umfasst einen der Länge nach verlaufenden Schalldurchgang 221, der mehrere Abschnitte mit verschiedenen Durchgangsquerschnitten aufweist. Durch die Anordnung der Abschnitte, die Wahl geeigneter Durchgangsquerschnitte und die Wahl der Abschnittslängen können die gewünschten Dämpfungseigenschaften des Filters 22 erreicht werden.

Fig. 8 zeigt ein zweites Ausführungsbeispiel eines Filters 23, wie es in einem Filtereinsatz 2 des erfindungsgemässen Gehörschutzes eingesetzt sein kann. Entsprechend dem Filter 22 aus Fig. 7 umfasst das Filter 23 einen der Länge nach verlaufenden Schalldurchgang 231 mit mehreren Abschnitten verschiedenen Durchgangsquerschnitts. Zusätzlich ist im Schalldurchgang 231 ein Draht 232 und am einen Ende des Schalldurchgangs 231 ein Deckel 233 angeordnet, welche zusammen die Filter- und Dämpfungseigenschaften in geeigneter Weise festlegen.

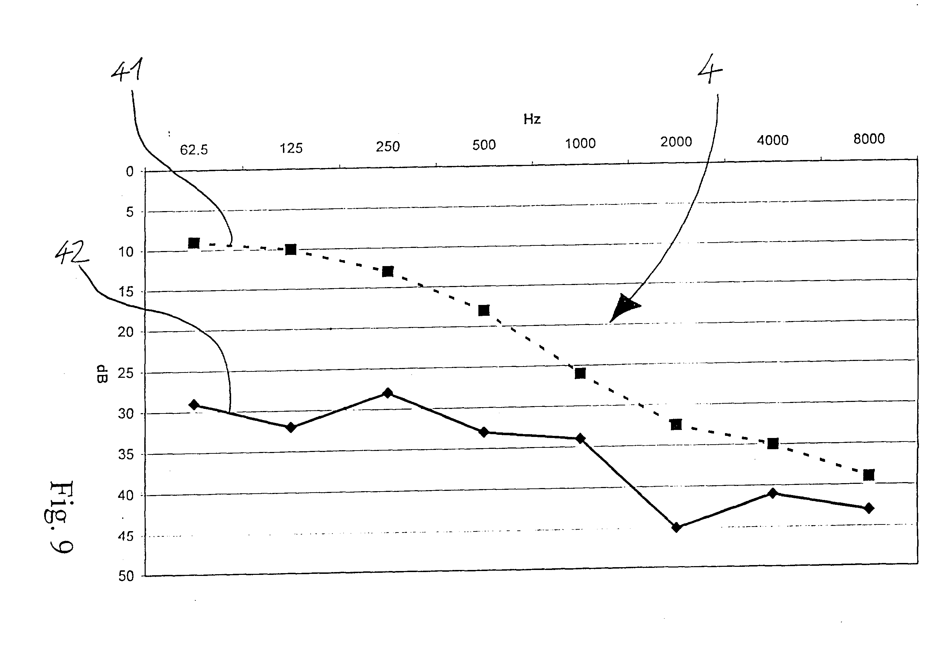

In Fig. 9 ist eine graphische Darstellung von Schalldämpfungskurven 4 zweier Ausführungsbeispiele von Filtern eines erfindungsgemässen Gehörschutzes gezeigt. Auf der Abszisse ist die Schallfrequenz logarithmisch in Hertz [Hz] aufgetragen und auf der Ordinate die Schalldämpfung in Dezibel [dB]. Beide Schalldämpfungskurven 41, 42 repräsentieren Filter 22 bzw. 23, die im allgemeinen Schall hoher Frequenz stärker dämpfen als solchen niedriger Frequenz. Im Vergleich zu Schalldämpfungskurve 41 stellt Schalldämpfungskurve 42 über alle dargestellten Frequenzen eine grössere Schalldämpfung dar, wobei der Unterschied mit zunehmender Schallfrequenz abnimmt. Die Schalldämpfungskurven 4 zeigen, dass zwei verschiedene Filter 22 bzw. 23 erheblich unterschiedliche Schalldämpfungseigenschaften aufweisen können und deshalb für unterschiedliche Einsatzzwecke vorgesehen sind.

Zu den vorbeschriebenen erfindungsgemässen Ausgestaltungen des Gehörschutzes sind weitere konstruktive Variationen realisierbar. Hier ausdrücklich erwähnt sei noch, dass die Otoplastiken 1 typischerweise paarweise mit jeweils einer Otoplastik 1 für den linken bzw. den rechten Gehörgang des Benutzers bereitgestellt werden können. Die halbringförmigen Fortsätze 11 der beiden Otoplastiken 1 eines Paares können über eine Schnur miteinander verbunden sein, sodass der Gehörschutz vor einem Verlust gesichert ist.

## Patentansprüche

1. Gehörschutz mit einer in den Gehörgang eines Benutzers einsetzbaren Otoplastik (1) und einem in die Otoplastik (1) in einer vordefinierten Position eingesetzten Filtereinsatz (2), **dadurch gekennzeichnet, dass** der Filtereinsatz (2) und die Otoplastik (1) so ausgebildet sind, dass der Filtereinsatz (2) durch den Benutzer selbst aus der Otoplastik (1) entfernbar und wieder korrekt in die vordefinierte Position einsetzbar ist.

2. Gehörschutz nach Anspruch 1, bei welchem der Filtereinsatz (2) eine Filterhülse (20; 21) umfasst, die aus einem Material hergestellt ist, z. B. aus Polyethylen niedriger Dichte, welches relativ zu dem Material aus dem die Otoplastik (1) hergestellt ist, z. B. Polyacryl, quasi nicht verformbar ist, sowie ein in die Filterhülse (20; 21) eingesetztes Filter.

3. Gehörschutz nach einem der vorangehenden Ansprüche, bei welchem der Filtereinsatz (2) einen Rand (201; 211) umfasst, der nach aussen hin absteht.

4. Gehörschutz nach einem der vorangehenden Ansprüche, bei welchem der Filtereinsatz (2) einen kegelstumpfförmigen Bereich (202; 212) aufweist.

5. Gehörschutz nach Anspruch 3 und 4, bei welchem der Rand (201; 211) des Filtereinsatzes (2) am distalen Ende des kegelstumpfförmigen Bereichs (202; 212) angeordnet ist.

6. Gehörschutz nach einem der vorangehenden Ansprüche, bei welchem die Otoplastik (1) individuell dem Gehörgang seines Benutzers angepasst ist.

7. Gehörschutz nach einem der vorangehenden Ansprüche, bei welchem die Otoplastik (1) lediglich den Eingangsbereich des Gehörgangs abdeckt.

8. Gehörschutz nach einem der vorangehenden Ansprüche, bei welchem die Otoplastik (1) Mittel (11) zum Einsetzen des Gehörschutzes in den Gehörgang bzw. zum Entfernen des Gehörschutzes aus dem Gehörgang umfasst.

9. Filtereinsatz (2) zum Einsetzen in die Otoplastik (1) eines Gehörschutzes nach einem der vorangehenden Ansprüche mit einem kegelstumpfförmigen Bereich (202; 212) und einem am distalen Ende des kegelstumpfförmigen Bereichs (202; 212) angeordneten Rand (201; 211), der von dem kegelstumpfförmigen Bereich (202; 212) nach aussen hin absteht.

10. Filtereinsatz (2) nach Anspruch 8 mit einer Filterhülse (20; 21), z. B. aus Polyethylen niedriger Dichte, und ein in die Filterhülse (20; 21) eingesetztes Filter (22; 23).

11. Filtereinsatzset, das mindestens zwei Filtereinsätzen entsprechend Anspruch 8 oder 9 mit unterschiedlichen Dämpfungseigenschaften umfasst.
